# EUROPEAN PATENT APPLICATION

(11) **EP 3 726 219 A1**
(43) Date of publication of application: **21.10.2020**
(21) Application number: 18892688.5
(22) Date of filing: 21.12.2018
(51) Int. Cl.: G01N 33/569, G01N 1/40

(54) **METHOD FOR DIAGNOSING TUBERCULOSIS**

(30) Priority: 22.12.2017 KR 20170178159
(71) Applicant: Sugentech, Inc., Daejeon 34025 (KR); THE INDUSTRY & ACADEMIC COOPERATION IN CHUNGNAM NATIONAL UNIVERSITY (IAC), Daejeon 34134 (KR)
(72) Inventor: KIM, Eunkyung, Daejeon 34119 (KR); LEE, Sunhee, Daejeon 34022 (KR); YOO, Seungbum, Daejeon 35263 (KR); SOHN, Mi-Jin, Daejeon 34076 (KR); KIM, Hwa-Jung, Daejeon 34962 (KR); KIM, Paul, Daejeon 34329 (KR)
(74) Representative: Laufhütte, Dieter
(86) International application number: PCT/KR2018/016434
(87) International publication number: WO 2019/125034

(57) **Abstract**

A method for diagnosing tuberculosis of the present invention can improve the reactivity of tuberculous antigens and detection antibodies by increasing free tuberculous antigens in a sample through a pretreatment step in which the sample of a subject is treated with an acidic material such that the antigens are dissociated from tuberculous antigen complexes, thereby enabling a point-of-care test for tuberculosis and being immediately utilizable as a fast and simple detection method for a point-of-care test.

## Description

### [Technical Field]

The present invention relates to a method capable of implementing a quick point-of-care test for tuberculosis by increasing a ratio of a free tuberculosis antigen which may be used for diagnosis of tuberculosis.

### [Background Art]

Tuberculosis which is an infectious disease caused by an infection with mycobacterium tuberculosis is known worldwide as a disease with a high incidence and a high mortality rate. In a case where tuberculosis is not diagnosed and treated at an early stage, a patient is likely to die. Therefore, it is required to conduct studies on a method for diagnosing tuberculosis.

Various methods for diagnosing tuberculosis have been known. In a case of a tuberculin skin test (TST), sensitivity is high and the test is carried out at a low cost, but reliability of tuberculosis diagnosis is low due to low specificity. A mycobacterium tuberculosis culture method is the most reliable screening method, but it is highly dangerous due to separation and culture of mycobacterium tuberculosis, and it takes a very long time to obtain a diagnosis result due to a long culture period. Recently, a method for reducing a culture period using a liquid medium has been introduced, but an additional definite diagnosis test for determining atypical mycobacterium tuberculosis is required. A nucleic acid amplification method is advantageous in terms of quick diagnosis, high sensitivity, and determination of drug resistance; however, since expensive equipment and human resources are required, it is difficult to use in low-income countries where there are high demands for a tuberculosis diagnosis product.

For an early diagnosis and treatment of tuberculosis, a method for diagnosing tuberculosis capable of implementing a point-of-care test (POCT) is required. In a smear stain which is one of methods of POCT for tuberculosis using a microscope, trained professionals and laboratories are required, and diagnosis accuracy is low due to low sensitivity. A serological tuberculosis diagnosis method is a method for diagnosing an antibody produced by infection with mycobacterium tuberculosis by collecting blood or serum of a patient. A quick and inexpensive antibody diagnosis product is currently being commercialized. In the case of this product, a diagnosis result may be confirmed within minutes and professionals are not required, but it is difficult to distinguish latent tuberculosis from active tuberculosis and sensitivity is very low. A diagnosis method through a mycobacterium tuberculosis antigen is a method for diagnosing tuberculosis by detecting a mycobacterium tuberculosis antigen in sputum, serum, urine, or pituitary gland, and various products using the method are provided, but culture of mycobacterium tuberculosis is required or accuracy, sensitivity, and the like are low in some cases. An example of the method for diagnosing tuberculosis by detecting a mycobacterium tuberculosis antigen includes Korean Patent No. 10-1524109.

In order to solve the problems of the method for diagnosing tuberculosis, the present invention provides a method capable of implementing a simple and quick point-of-care test for tuberculosis by dissociating antigen complexes produced in a patient infected with tuberculosis in a simple manner and using free antigens increased by the dissociation of the antigen complexes.

### [Related Art Document]

### [Patent Document]

Korean Patent No. 10-1524109

### [Disclosure]

### [Technical Problem]

An object of the present invention is to provide a method capable of implementing a point-of-care test for tuberculosis by dissociating antigens from antigen complexes included in a specimen collected from a subject for a point-of-care test for tuberculosis to increase free antigens, and using the increased free antigens.

### [Technical Solution]

In one general aspect, there is provided a method for diagnosing tuberculosis that treats a sample collected from a subject with an acidic substance, and then treats antibodies for mycobacterium tuberculosis secreted antigens.

In another general aspect, a kit for testing for tuberculosis includes: an acidic reagent for dissociating antigens from tuberculosis antigen complexes included in a sample collected from a subject; a neutralizing reagent for neutralizing the dissociated antigens; and a solid phase to which antibodies are immobilized.

### [Advantageous Effects]

The present invention may provide a tuberculosis point-of-care test method with high accuracy and sensitivity. The method for diagnosing tuberculosis according to the present invention may be simply and quickly performed, and may be immediately applied to a point-of-care test.

### [Description of Drawings]

FIG. 1 illustrates a procedure of a method for diagnosing tuberculosis according to the present invention (tuberculosis (TB) patient serum).
FIG. 2 illustrates a lamination structure of an immunochromatography strip used in an embodiment of the present invention.
FIG. 3 illustrates tuberculosis diagnosis results according to the method for diagnosing tuberculosis according to the present invention.

### [Best Mode]

Hereinafter, the present invention will be described in detail. The terms used herein should be interpreted as generally understood by those skilled in the art unless otherwise defined. The drawings and embodiments herein are provided for those skilled in the art to easily understand and practice the present invention. In the drawings and the embodiments, contents that may obscure the gist of the invention may be omitted. The present invention is not limited to the drawings and embodiments.

The present invention relates to a method capable of implementing a point-of-care test for tuberculosis, and may provide a method for diagnosing tuberculosis that treats a sample collected from a subject with an acidic substance, and then treats antibodies for mycobacterium tuberculosis secreted antigens, or a method of providing information for tuberculosis diagnosis.

Specifically, the present invention may provide a method for diagnosing tuberculosis, the method including: collecting a sample (specimen) from a subject; treating the sample with an acidic substance; neutralizing the sample; and treating antibodies for mycobacterium tuberculosis specific antigens.

In the present invention, the subject includes a human or an animal that is already infected with tuberculosis or is likely to be infected with tuberculosis, and may refer to all targets for the tuberculosis diagnosis.

In the present invention, the sample (or specimen) of the subject is preferably one or more selected from the group consisting of blood (whole blood, plasma, or serum), urine, sputum, saliva, joint fluid, oral mucosal transudate, and pituitary gland, but is not limited thereto. The sample of the subject may include all samples in which mycobacterium tuberculosis antigen complexes produced by mycobacterium tuberculosis may be present.

In the present invention, the acidic substance may collectively include substances for dissociating mycobacterium tuberculosis antigens from the mycobacterium tuberculosis antigen complexes included in the sample of the subject. The acidic substance is one or more selected from the group consisting of a glycine buffer, a citrate buffer, and an acetate buffer, and the glycine buffer is preferable, but the present invention is not limited thereto.

Any acidic substance may be used without a particular limitation as long as it may dissociate the mycobacterium tuberculosis antigens from the mycobacterium tuberculosis antigen complexes. In the present invention, the mycobacterium tuberculosis antigen complex may refer to a complex that may be formed by binding of a mycobacterium tuberculosis antigen to a biomolecule, a compound, or an artificial substance. Representative examples thereof include an antigen-antigen complex and an antigen-antibody complex, but are not limited thereto.

In the present invention, a pH of the acidic substance is 1.0 to 6.0, 1.0 to 5.5, 1.0 to 5.0, 1.0 to 4.5, 1.0 to 4.0, 2.0 to 6.0, 2.0 to 5.5, 2.0 to 5.0, or 2.0 to 4.5, and is preferably 2.0 to 4.0. In the present invention, a concentration of the acidic substance is 0.05 to 2.0 M, 0.05 to 1.5 M, 0.05 to 1.0 M, 0.05 to 0.5 M, 0.05 to 0.1 M, 0.1 to 2 M, 0.1 to 1.5 M, 0.1 to 1.0 M, 0.1 to 0.9 M, 0.1 to 0.8 M, 0.1 to 0.7 M, 0.1 to 0.6 M, or 0.1 to 0.5 M, and is preferably 0.05 to 2.0. Since a binding affinity of an antigen-antigen or an antigen-antibody may vary depending on a type of antigen complex, and the concentration of the acidic substance may vary depending on a type of antigen complex and neutralizing reagent.

An example of the antigen-antigen complex includes an Ag85 complex such as Ag85A, Ag85B, or Ag85C. It is known that the Ag85 complex binds to fibronectin and elastin protein of a host cell to contribute to infection activity of mycobacterium tuberculosis. Another example of the antigen-antigen complex includes CFP10/ESAT6 formed by a heterodimer composed of CFP-10 and ESAT6 that are CFP-10 protein and ESAT6 protein, respectively, and expressed together in an RD1 gene. It is known that CFP10/ESAT6 contributes to pathogenicity of mycobacterium tuberculosis.

The antigen-antibody complex is an immune complex formed in body fluids of a tuberculosis patient, such as blood, and may include a complex formed by expression of IgG, IgA, or IgM specific to the mycobacterium tuberculosis secreted antigen.

In the present invention, the mycobacterium tuberculosis secreted antigen dissociated from the mycobacterium tuberculosis antigen complex may be one or more selected from the group consisting of MPT64, a 6-kDa early secreted antigenic target (ESAT6), a 10-kDa culture filtrate protein (CFP10), HspX (14kDa), 38kDa protein, LAM, and an antigen 85 complex (Ag85), but is not limited thereto.

In the present invention, a specific antibody for the mycobacterium tuberculosis antigen may be one or more selected from the group consisting of a monoclonal (or polyclonal) anti-CFP10 antibody, a monoclonal (or polyclonal) anti-Ag85A antibody, a monoclonal (or polyclonal) anti-Ag85B antibody, a monoclonal (or polyclonal) anti-Ag85C antibody, a monoclonal (or polyclonal) anti-ESAT6 antibody, a monoclonal (or polyclonal) anti-HspX(14kDa) antibody, a monoclonal (or polyclonal) anti-MPT64 antibody, a monoclonal (or polyclonal) anti-38kDa antibody, and a monoclonal (or polyclonal) anti-LAM antibody, but is not limited thereto.

More specifically, the method for diagnosing tuberculosis according to the present invention may be performed by the following methods.

The method for diagnosing tuberculosis according to the present invention may include: collecting a sample from a subject; treating an acidic substance to dissociate antigens from tuberculosis antigen complexes included in the sample; treating a neutralizing reagent to neutralize the sample including the dissociated antigens or the dissociated antigens; and treating antibodies capable of binding specifically to the dissociated antigens.

The collecting of the sample from the subject may be performed by a known detection method depending on a type of sample (sputum, urine, blood, or the like), and is not particularly limited thereto.

The antigen complexes formed by the mycobacterium tuberculosis antigens may be present in the sample collected from the subject. The treating of the acidic substance is performed to dissociate the antigens from the antigen complexes. When the sample is treated with the acidic substance, the antigens are dissociated from the antigen complexes, and free antigens in the sample are increased. An antigen-antibody reaction caused by the specific antibody for the mycobacterium tuberculosis antigen to be treated is also increased in accordance with the increase of the free antigens, which may increase accuracy and sensitivity of the tuberculosis diagnosis.

The sample collected from the subject is treated with the acidic substance, and the treated sample is subjected to a neutralization step to neutralize the acidic sample or the antigens included in the sample. The neutralization step is required to provide an environment suitable for reacting the free antigens and the antibodies with each other. A neutralizing reagent for the neutralization is not particularly limited as long as it is a basic substance, and may be appropriately used depending on a previously used acidic substance, a type of antigen, a type of antibody to treat, and the like. As the neutralizing reagent, for example, one or more selected from the group consisting of tris-[hydroxymethyl]-aminomethane (TRIS), carbonate, and borate may be used, but the present invention is not limited thereto. In the reaction, a volume ratio of the sample to the neutralizing reagent is preferably 1:1 to 1:10, but is not limited thereto. The volume ratio may be appropriately adjusted depending on a reagent to be used and a degree of acidity of the sample.

In the present invention, a pH of the neutralizing reagent is 8.0 to 10.5, 8.0 to 11.0, 9.0 to 10.0, 9.0 to 10.5, or 9.0 to 11.0, and preferably 8.0 to 10.0.

In the present invention, a concentration of the neutralizing reagent is 0.02 to 0.5 M, 0.02 to 0.4 M, 0.02 to 0.3 M, 0.02 to 0.2 M, 0.03 to 0.5 M, 0.03 to 0.4 M, 0.03 to 0.3 M, 0.03 to 0.2 M, 0.04 to 0.5 M, 0.04 to 0.4 M, 0.04 to 0.3 M, 0.04 to 0.2 M, 0.05 to 0.5 M, 0.05 to 0.4 M, 0.05 to 0.3 M, 0.05 to 0.2 M, 0.06 to 0.5 M, 0.06 to 0.4 M, 0.06 to 0.3 M, 0.06 to 0.2 M, 0.07 to 0.5 M, 0.07 to 0.4 M, 0.07 to 0.3 M, 0.07 to 0.2 M, 0.08 to 0.5 M, 0.08 to 0.4 M, 0.08 to 0.3 M, 0.08 to 0.2 M, 0.09 to 0.5 M, 0.09 to 0.4 M, 0.09 to 0.3 M, 0.09 to 0.2 M, 0.1 to 0.5 M, 0.1 to 0.4 M, 0.1 to 0.3 M, or 0.1 to 0.2 M. The concentration of the neutralizing reagent may vary depending on an acidic substance to be used and a type of tuberculosis antigen complex.

According to an embodiment of the present invention, in order to detect Ag85 in the tuberculosis antigens, in a case where 0.05 to 2.0 M glycine is used as the acidic substance for treatment, when 0.1 to 0.5 M TRIS is used as the neutralizing reagent for the treatment, an effect of detecting Ag85 may be significantly increased. According to another embodiment of the present invention, in order to detect CFP10 in the tuberculosis antigens, in a case where 0.05 to 1.0 M glycine is used as the acidic substance for treatment, when 0.02 to 0.2 M TRIS is used as the neutralizing reagent for the treatment, an effect of detecting CFP10 may be significantly increased. According to still another embodiment of the present invention, in order to detect ESAT6 in the tuberculosis antigens, in a case where 0.05 to 1.0 M glycine is used as the acidic substance for treatment, when 0.02 to 0.2 M TRIS is used as the neutralizing reagent for the treatment, an effect of detecting ESAT6 may be significantly increased.

The sample is treated with the antibodies after the neutralization step or together with the neutralizing reagent treatment, such that an antigen-antibody reaction of the antibody with the mycobacterium tuberculosis antigen including the free antigens included in the sample may be induced. It may be appropriately selected depending on a type of mycobacterium tuberculosis antigen which is a detection target of the antibody, and is not particularly limited. The antibody is a detection antibody for detecting an antigen, and induces a competition reaction of the antigen with other proteins forming a complex. The antibody is preferably a labeled antibody to which an indicator is bound, but is not limited thereto. The labeled antibody may be labeled by an antibody-indicator conjugant. Various dyeing materials such as a gold nano-particle, an enzyme such as peroxidase, a fluorescent substance, a latex bead, and a cellulose nano-bead may be used as the indicator, but the present invention is not limited thereto.

After the antibody treatment, an immune detection step may be performed to detect binding between the antigen and the antibody in the sample. An immune detection method is not particularly limited, and tuberculosis may be diagnosed through the binding between the antibody and the mycobacterium tuberculosis antigen by various known immune detection methods. According to an embodiment of the present invention, after an antigen-detection antibody in a neutralization buffer is reacted with another antibody immobilized to immunochromatography or a microplate, tuberculosis infection may be finally confirmed through cleaning or a color reaction.

The present invention may provide a kit for tuberculosis diagnosis capable of implementing the method for diagnosing tuberculosis and being used for a point-of-care test for tuberculosis.

The kit for tuberculosis diagnosis according to the present invention may include: an acidic reagent for dissociating antigens from tuberculosis antigen complexes included in a sample collected from a subject; a neutralizing reagent for neutralizing the dissociated antigens; and a solid phase to which antibodies are immobilized.

In the present invention, the kit for tuberculosis diagnosis may be manufactured into an immunochromatography kit, an enzyme-linked immunosorbent assay (ELISA) kit, or a competition assay kit depending on types of solid phase to which antibodies are immobilized, antibody which is used for an antigen-antibody reaction, enzyme, and labeling substance (radioactive substance, fluorescent substance, or the like).

The kit for tuberculosis diagnosis according to the present invention may further include an antibody which is specific to the tuberculosis antigen or is capable of binding to an immobilized antibody.

Hereinafter, examples for implementing the present invention will be described. It should be noted that each of the examples correspond to one example for implementing the present invention, and the present invention is not to be construed as limited to the examples.

### [Example 1]

### (1) Tuberculosis Diagnosis Using Immunochromatography Method

A plasma or serum sample collected from a subject infected with tuberculosis was mixed with 1.0 M glycine with a pH of 2.0 to 4.0 used as an acidic substance in a volume ratio (sample/acidic substance) of 1/10, and the mixture was subjected to a pretreatment within 30 minutes to allow a reaction to proceed.

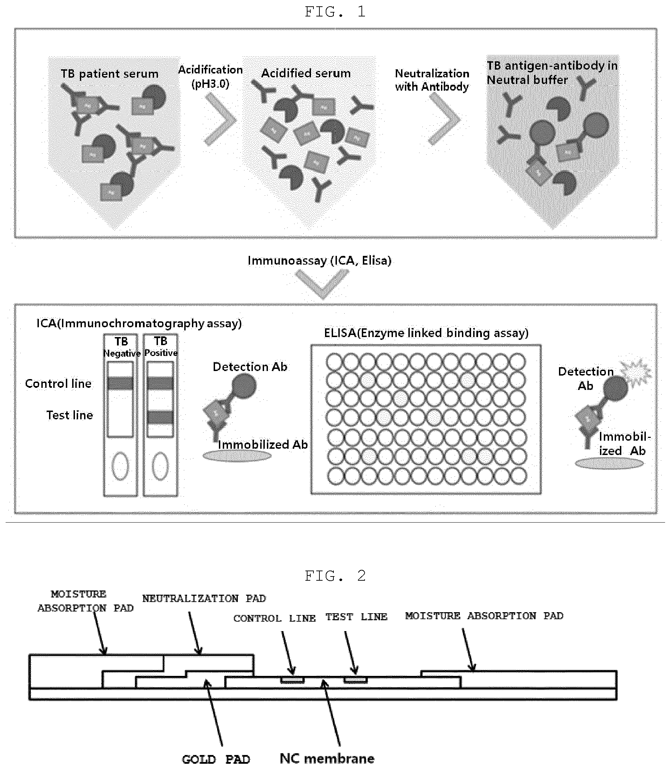

For tuberculosis diagnosis through detection of free antigens included in the sample reacted with the glycine, an immunochromatography tester was prepared as follows.
- Antibody immobilization: antibodies were dispensed on an NC membrane in a line form, and the dispensed antibodies were dried and immobilized.
- Neutralization pad configuration: a neutralization pad was configured by preparing a buffer treated with 100 mM Tris-HCL with a pH 8.0, 0.5 to 2.0% casein, a 0.5 to 2% surfactant (Tween20, Triton X-100), and 20 to 500 mM NaCl, wetting glass fiber with the buffer, and drying the glass fiber.
- Neutralization buffer: a buffer treated with 100 mM Tris-HCL with a pH 8.0 or more, 0.5 to 2.0% casein, a 0.5 to 2% surfactant (Tween20, Triton X-100), and 20 to 500 mM NaCl was prepared.
- Gold-antibody conjugate preparation: colloidal gold having a size of 40 to 60 nm was reacted with an anti-tuberculosis antigen monoclonal antibody derived from a mouse at 1 to 10 µg/ml, and casein or bovine serum albumin (BSA) was treated with a blocking agent at 0.1 to 1.0% and washed, thereby obtaining a purified anti-tuberculosis gold-antibody conjugate.
- Conjugate pad preparation: a pad was configured by treating the anti-tuberculosis gold-antibody conjugate with a phosphate buffer in which sucrose and BSA were added at an appropriate concentration, filtering the treated anti-tuberculosis gold-antibody conjugate by a 0.45 µm filter, wetting a conjugate pad with the filtered anti-tuberculosis gold-antibody conjugate, and drying the conjugate pad.
- Immunochromatography strip preparation: as illustrated in FIG. 2, an immunochromatography strip was prepared using an antibody immobilization NC membrane, a neutralization pad, and a conjugate pad treated with the gold-antibody conjugate.

### (2) Tuberculosis Diagnosis Using Immunochromatography Method

A plasma or serum sample collected from a subject infected with tuberculosis was mixed with 1.0 M glycine with a pH of 2.0 to 4.0 used as an acidic substance in a volume ratio (sample/acidic substance) of 1/10, and the mixture was subjected to a pretreatment within 30 minutes to allow a reaction to proceed.

For tuberculosis diagnosis through detection of free antigens included in the sample reacted with the glycine, an immunochromatography tester was prepared as follows.
- Antibody immobilization: antibodies were dispensed on an NC membrane in a line form, and the dispensed antibodies were dried and immobilized.
- Neutralization antibody: a buffer treated with 100 mM Tris-HCL with a pH 8.0, 0.5 to 2.0% casein, a 0.5 to 2% surfactant (Tween20, Triton X-100), and 20 to 500 mM NaCl was prepared, colloidal gold having a size of 40 to 60 nm was reacted with an anti-tuberculosis antigen monoclonal antibody derived from a mouse at 1 to 10 µg/ml, and casein or bovine serum albumin (BSA) was treated with a blocking agent at 0.1 to 1.0% and washed, a purified anti-tuberculosis gold-antibody conjugate was added, and components such as PEG, dextran, PVA, sucrose, trehalose, and mannitol used as a stabilizer were added in an appropriate amount. As described above, a liquid neutralization antibody or a dried neutralization antibody obtained by additionally freeze-drying the liquid neutralization antibody for 16 to 48 hours was produced.
- Immunochromatography strip preparation: an immunochromatography strip having the same structure as that illustrated in FIG. 2 was prepared using an antibody immobilization NC membrane and glass fiber or cellulose pads for chromatography.

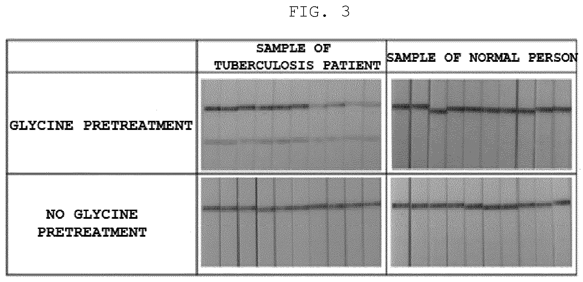

On the immunochromatography strip prepared as described above, a sample of a subject subjected to a pretreatment with glycine itself was treated, or the sample of the subject subjected to the pretreatment and the neutralization buffer or the sample of the subject subjected to the pretreatment and the neutralization antibody were mixed with each other in an appropriate ratio and were treated, and results of tuberculosis antigen detection were confirmed (FIG. 3).

For detection of CFP-10 which is a tuberculosis antigen, an anti-CFP-10 antibody was used as an antibody.

As a result of the tuberculosis antigen detection, it could be confirmed that the tuberculosis antigens were detected in the sample subjected to the pretreatment with glycine because a distinct band appeared at a position of a test line of the immunochromatography strip. However, in the sample subjected to no pretreatment with glycine, a degree of detection of the tuberculosis antigen was very low because a band hardly appeared at the position of the test line.

Even in a case where an anti-Ag85 antibody was used to detect Ag85 with a tuberculosis antigen (referring to Examples 2 to 34), in the sample subjected to the pretreatment with glycine, detection of the tuberculosis antigens was confirmed because a distinct band appeared at the position of the test line of the immunochromatography strip, whereas, in the sample subjected to no pretreatment with glycine, the degree of detection of the tuberculosis antigen was very low because a band hardly appeared at the position of the test line.

These results show that it is possible to accurately diagnose tuberculosis by the method for diagnosing tuberculosis according to the present invention even in a patient who is in a situation where it is difficult to accurately determine tuberculosis by a general antigen-antibody reaction (a patient with insufficient detection antigens from the tuberculosis antigen complex).

In addition, the immunochromatography strip may be simply prepared, and a preparation cost thereof is low, and thus the immunochromatography strip may be used by immediately applying the method diagnosing tuberculosis according to the present invention to a point-of-care test.

### [Examples 2 to 34]

Detection of the tuberculosis antigen was performed in the same manner as that of Example 1. The used acidic substance, neutralization reagent, and antibody, and whether the tuberculosis antigen is detected are shown in Tables 1 to 4.

Whether the tuberculosis antigen was detected was confirmed through the number of bands that appeared (appeared by binding of the tuberculosis antigens dissociated from the tuberculosis antigen complexes to the used antibodies) at the test line on each of 20 immunochromatography strips. As a result of calculation according to the following equation, detection intensity was graded "high" when the result was 60% or more, the detection intensity was graded "low" when the result was 20% or more and less than 60%, and the detection intensity was graded "not detected" when the result was less than 20%.

Detection intensity = [number of immunochromatography strips with test line at which band appeared/total number of immunochromatography strips] ^{∗} 100

Since whether the result may be immediately determined with the naked eye is important in order to be used for a point-of-care test, whether the band appeared at the test line was determined with the naked eye.

**[Table 1]**

| | | Example 2 | Example 3 | Example 4 | Example 5 | Example 6 | Example 7 | Example 8 | Example 9 | Example 10 |
|---|---|---|---|---|---|---|---|---|---|---|
| Sample collection site | | Serum (10) Plasma (10) | Serum (10) Plasma (10) | Serum (10) Plasma ( 10) | Serum (10) Plasma( 10) | Serum (10) Plasma( 10) | Serum (10) Plasma( 10) | Serum (10) Plasma( 10) | Serum (10) Plasma( 10) | Serum (10) Plasma( 10) |
| Acidic substance | Type | Glycine | Glycine | Glycine | Glycine | Glycine | Glycine | Glycine | Glycine | Glycine |
| | Concentration | 0.05 M | 0.1 M | 0.5 M | 0.5 M | 1 M | 1 M | 1.5 M | 2 M | 2 M |
| | pH | 3.0 | 3.0 | 3.0 | 3.0 | 3.0 | 3.0 | 3.0 | 3.0 | 3.0 |
| Neutralizing reagent | Type | TRIS | TRIS | TRIS | TRIS | TRIS | TRIS | TRIS | TRIS | TRIS |
| | Concentration | 0.05 M | 0.05M | 0.1 M | 0.2 M | 0.1 M | 0.2 M | 0.3 M | 0.5 M | 0.8 M |
| | pH | 9.0 | 9.0 | 9.0 | 9.0 | 9.0 | 9.0 | 9.0 | 9.0 | 9.0 |
| Reaction ratio (v/v) of acidic substance/neutralizing reagent | | 1:1 | 1:1 | 1:1 | 1:1 | 1:1 | 1:1 | 1:1 | 1:1 | 1:1 |
| Antibody | | Anti-Ag85B | Anti-Ag85B | Anti-Ag85B | Anti-Ag85B | Anti-Ag85B | Anti-Ag85B | Anti-Ag85B | Anti-Ag85B | Anti-Ag85B |
| Detection intensity | | Not detected | Low | High | High | High | High | High | High | Low |

From the results shown in Table 1, it was confirmed that when the concentration of glycine was 0.5 to 2.0 M and the concentration range of TRIS was 0.1 to 0.5 M, Ag85B was effectively dissociated and detected from the tuberculosis-antigen complex.

**[Table 2]**

| | | Example 11 | Example 12 | Example 13 | Example 14 | Example 15 | Example 16 | Example 17 | Example 18 | Example 19 |
|---|---|---|---|---|---|---|---|---|---|---|
| Sample collection site | | Serum (10) Plasma (10) | Serum (10) Plasma (10) | Serum (10) Plasma (10) | Serum (10) Plasma (10) | Serum (10) Plasma (10) | Serum (10) Plasma (10) | Serum (10) Plasma (10) | Serum (10) Plasma (10) | Serum (10) Plasma (10) |
| Acidic substance | Type | Glycine | Glycine | Glycine | Glycine | Glycine | Glycine | Glycine | Glycine | Glycine |
| | Concentration | 0.02 M | 0.05 M | 0.05 M | 0.1 M | 0.1 M | 0.5 M | 1 M | 1 M | 2 M |
| | pH | 3.0 | 3.0 | 3.0 | 3.0 | 3.0 | 3.0 | 3.0 | 3.0 | 3.0 |
| Neutralizing reagent | Type | TRIS | TRIS | TRIS | TRIS | TRIS | TRIS | TRIS | TRIS | TRIS |
| | Concentration | 0.01M | 0.01 M | 0.02 M | 0.02 M | 0.05 M | 0.1 M | 0.2 M | 0.4 M | 0.4 M |
| | pH | 9.0 | 9.0 | 9.0 | 9.0 | 9.0 | 9.0 | 9.0 | 9.0 | 9.0 |
| Reaction ratio (v/v) of acidic substance/neutralizing reagent | | 1:1 | 1:1 | 1:1 | 1:1 | 1:1 | 1:1 | 1:1 | 1:1 | 1:1 |
| Antibody | | Anti-CFP10 | Anti-CFP10 | Anti-CFP10 | Anti-CFP10 | Anti-CFP10 | Anti-CFP10 | Anti-CFP10 | Anti-CFP10 | Anti-CFP10 |
| Detection intensity | | Not detected | Low | High | High | High | High | High | Low | Not detected |

From the results shown in Table 2, it was confirmed that when the concentration of glycine was 0.05 to 1.0 M and the concentration range of TRIS was 0.02 to 0.2 M, CFP10 was effectively dissociated and detected from the tuberculosis-antigen complex.

**[Table 3]**

| | | Example 20 | Example 21 | Example 22 | Example 23 | Example 24 | Example 25 | Example 26 | Example 27 | Example 28 |
|---|---|---|---|---|---|---|---|---|---|---|
| Sample collection site | | Serum (10) Plasma (10) | Serum (10) Plasma (10) | Serum (10) Plasma (10) | Serum (10) Plasma (10) | Serum (10) Plasma (10) | Serum (10) Plasma (10) | Serum (10) Plasma (10) | Serum (10) Plasma (10) | Serum (10) Plasma (10) |
| Acidic substance | Type | Glycine | Glycine | Glycine | Glycine | Glycine | Glycine | Glycine | Glycine | Glycine |
| | Concentratio n | 0.02 M | 0.05 M | 0.05 M | 0.1 M | 0.1 M | 0.5 M | 1 M | 1 M | 2 M |
| | pH | 3.0 | 3.0 | 3.0 | 3.0 | 3.0 | 3.0 | 3.0 | 3.0 | 3.0 |
| Neutralizin g reagent | Type | TRIS | TRIS | TRIS | TRIS | TRIS | TRIS | TRIS | TRIS | TRIS |
| | Concentratio n | 0.01 M | 0.01 M | 0.02 M | 0.02 M | 0.05 M | 0.1 M | 0.2 M | 0.4 M | 0.4 M |
| | pH | 9.0 | 9.0 | 9.0 | 9.0 | 9.0 | 9.0 | 9.0 | 9.0 | 9.0 |
| Reaction ratio (v/v) of acidic substance/ neutralizing reagent | | 1:1 | 1:1 | 1:1 | 1:1 | 1:1 | 1:1 | 1:1 | 1:1 | 1:1 |
| Antibody | | Anti-ESAT6 | Anti-ESAT6 | Anti-ESAT6 | Anti-ESAT6 | Anti-ESAT6 | Anti-ESAT6 | Anti-ESAT6 | Anti-ESAT6 | Anti-ESAT6 |
| Detection intensity | | Not detecte d | Low | High | High | High | High | High | Low | Not detecte d |

From the results shown in Table 3, it was confirmed that when the concentration of glycine was 0.05 to 1.0 M and the concentration range of TRIS was 0.02 to 0.2 M, ESAT6 was effectively dissociated and detected from the tuberculosis-antigen complex.

**[Table 4]**

| | | Example 29 | Example 30 | Example 31 | Example 32 | Example 33 | Example 34 |
|---|---|---|---|---|---|---|---|
| Sample collection site | | Serum (10) Plasma (10) | Serum (10) Plasma (10) | Serum (10) Plasma (10) | Serum (10) Plasma (10) | Serum (10) Plasma (10) | Serum (10) Plasma (10) |
| Acidic substance | Type | Glycine | Glycine | Glycine | Glycine | Glycine | Glycine |
| | Concentration | 0.1 M | 0.1 M | 0.1 M | 0.1 M | 0.1 M | 0.1 M |
| | pH | 3.0 | 3.0 | 3.0 | 3.0 | 3.0 | 3.0 |
| Neutralizing reagent | Type | Borate | Carbonate | Borate | Carbonate | Borate | Carbonate |
| | Concentration | 0.05 M | 0.05 M | 0.05 M | 0.05 M | 0.05 M | 0.05 M |
| | pH | 9.0 | 9.0 | 9.0 | 9.0 | 9.0 | 9.0 |
| Reaction ratio (v/v) of acidic substance/ neutralizing reagent | | 1:1 | 1:1 | 1:1 | 1:1 | 1:1 | 1:1 |
| Antibody | | Anti-Ag85B | Anti-Ag85B | Anti-CFP10 | Anti-CFP10 | Anti-ESAT6 | Anti-ESAT6 |
| Detection intensity | | High | High | High | High | High | High |

From the results shown in Table 4, it was confirmed that even in a case where borate and carbonate were used as the neutralizing reagent, Ag85B, CFP10, and ESAT6 were also effectively dissociated and detected from the tuberculosis-antigen complex.

From the above results, it was confirmed that the tuberculosis antigens (CFP10, Ag85, and ESAT6) were effectively dissociated and detected from the tuberculosis-antigen complex by the acidic substance treatment and the neutralizing reagent treatment.

## Claims

1. A method for diagnosing tuberculosis, comprising:
treating an acidic substance to dissociate antigens from tuberculosis antigen complexes included in a sample collected from a subject;
treating a neutralizing reagent to neutralize the sample including the dissociated antigens or the dissociated antigens; and
treating antibodies capable of binding specifically to the dissociated antigens.

2. The method of claim 1, wherein the sample collected from the subject is selected from the group consisting of blood (whole blood, plasma, or serum), urine, sputum, saliva, joint fluid, oral mucosal transudate, and pituitary gland.

3. The method of claim 1, wherein the antigen is selected from the group consisting of MPT64, a 6-kDa early secreted antigenic target (ESAT6), 10-kDa culture filtrate protein (CFP10), an antigen 85 complex (Ag85), Ag85A, Ag85B, Ag85C, HspX, LAM, and 38kDa protein.

4. The method of claim 1, wherein the acidic substance is selected from the group consisting of a glycine buffer, a citrate buffer, and an acetate buffer.

5. The method of claim 1, wherein a pH of the acidic substance is 2.0 to 4.0.

6. The method of claim 1, wherein the treating of the neutralizing reagent is performed using a basic buffer solution or a basic buffer dried pad.

7. The method of claim 1, wherein the treating of the antibodies is performed using a dried pad with which an antibody-indicator conjugant is treated.

8. The method of claim 1, wherein the treating of the neutralizing reagent and the treating of the antibodies are simultaneously performed using a solution obtained by adding an antibody-indicator conjugant to a basic buffer solution, or a solid obtained by freeze-drying the solution obtained by adding the antibody-indicator conjugant to the basic buffer solution.

9. A kit for testing for tuberculosis, comprising:
an acidic reagent for dissociating antigens from tuberculosis antigen complexes included in a sample collected from a subject;
a neutralizing reagent for neutralizing the dissociated antigens; and
a solid phase to which antibodies are immobilized.

10. The kit of claim 9, wherein the kit is an immunochromatography kit or an enzyme-linked immunosorbent assay (ELISA) kit.
